# EUROPÄISCHE PATENTSCHRIFT

(11) **EP 2 417 857 B1**
(45) Veröffentlichungstag und Bekanntmachung des Hinweises auf die Patenterteilung: **09.11.2016**
(21) Anmeldenummer: 11177432.9
(22) Anmeldetag: 12.08.2011
(51) Int. Cl.: A23C 9/142, A23C 21/00, A23C 21/02, A23C 9/20, A61K 35/20

(54) **Verfahren zur Herstellung eines Kolostrum-Produktes und Vorrichtung zur Durchführung des Verfahrens**
Method for manufacturing a colostrum product and device for executing the method
Procédé destiné à la fabrication d'un produit de colostrum et dispositif destiné à l'exécution du procédé

(30) Priorität: 12.08.2010 DE 102010039278; 13.01.2011 DE 102011008579
(43) Veröffentlichungstag der Anmeldung: 15.02.2012
(73) Patentinhaber: LR Health & Beauty Systems GmbH, 59227 Ahlen (DE)
(72) Erfinder: Tiegs, Wilfried, 27308 Kirchlinteln (DE)
(74) Vertreter: Maikowski & Ninnemann Patentanwälte Partnerschaft mbB

(56) Entgegenhaltungen:
- EP-A2- 1 055 372
- WO-A1-98/00150
- WO-A1-2009/135306
- A ARGÜELLO: "Effects of refrigeration, freezing-thawing and pasteurization on IgG goat colostrum preservation", SMALL RUMINANT RESEARCH, Bd. 48, Nr. 2, 1. Mai 2003 (2003-05-01), Seiten 135-139, XP55013892, ISSN: 0921-4488, DOI: 10.1016/S0921-4488(02)00277-8

## Beschreibung

Die Erfindung betrifft ein Verfahren zur Herstellung eines Kolostrum-Produktes gemäß Anspruch 1 und eine Vorrichtung zur Durchführung des Verfahrens mit den Merkmalen des Anspruchs 15.

Kolostrum (auch Colostrum, Kolostralmilch, Biestmilch oder Erstmilch genannt) ist die erste Milch der ersten 4 bis 5 Tage nach der Geburt (post partum) eines frisch geborenen Tieres, wie eines Kalbes, eines Schafes oder einer Ziege.

Es hat sich herausgestellt, dass Kolostrum wertvolle Eigenschaften hat, so dass die Aufgabe besteht, besonders hochwertiges und haltbares Kolostrum herstellen zu können. Diese Aufgabe wird durch ein Verfahren mit den Merkmalen des Anspruchs 1 gelöst. Dabei wird
a) gefrorenes Rohkolostrum aufgetaut und auf eine Temperatur unterhalb von 10°C, vorzugsweise 4 bis 10°C aufgetaut wird, wobei das gefrorene Rohkolostrum vor und/oder während des Auftauens mechanisch in kürzester Zeit zerkleinert wird, und anschließend
b) durch eine Abtrennung ein Fettgehalt von weniger als 0,5% Massen-%, insbesondere von weniger als 0,2 Massen-% eingestellt wird, und dann
c) eine Ausfällung von Kasein durch eine Zugabe einer Protease in Form von des Lab-Enzyms Chymosin erfolgt und
d) die erhaltene Flüssigkeit mindestens einer Sterilfiltrationsstufe unterzogen wird.

Mit einem solchen Verfahren ist ein Kolostrum-Produkt erzielbar, dass eine geringe Keimbelastung und eine geringe Trübung aufweist.

Das Auftauen des gefrorenen Rohkolostrums erfolgt vorteilhafterweise innerhalb von 1 bis 5 Sekunden pro kg. Das gefrorene Rohkolostrum wird vor und / oder während des Auftauens mechanisch in kürzester Zeit mechanisch zerkleinert (gecrusht) und / oder eine kontinuierliche Vermahlung des gefrorenen Rohkolostrum-Eises zu Eispartikeln mit einem mittleren Durchmesser von ca. 1 cm erfolgt. Besonders vorteilhaft ist es, wenn das mechanische Zerkleinern zwischen 1 bis 5 Sekunden pro kg dauert, kontinuierlich stattfindet und das aufgetaute Rohkolostrum kontinuierlich abgezogen wird.

Das Brechen, d.h. die mechanische Zerkleinerung des Eises (Eiscrushen) bewirkt eine erhebliche Keimreduzierung. Damit werden die nachfolgenden Verfahrensschritte (z.B. Sterilfiltration) mit einer wesentlich geringeren Keimlast beaufschlagt, und auch das Fertigprodukt erzielt eine wesentlich geringere Keimzahl und ist dadurch wesentlich haltbarer.

In einer vorteilhaften Ausführungsform wird das gefrorene Rohkolostrum während der mechanischen Zerkleinerung aufgetaut. Das gefrorene Rohkolosturm wird dabei in Blöcken (z.B. 50 Blöcke à 3 Liter) in einen Eisbrecher (Eiscrusher) befördert und dort zerkleinert. Das Auftauen erfolgt vorteilhafterweise zu mindest teilweise während der mechanischen Zerkleinerung. Es ist ferner vorteilhaft, wenn die Einstellung des Fettgehaltes durch Zentrifugieren, insbesondere eine Milchzentrifuge, Schneckenzentrifuge und / oder Sedimentieren erreicht wird. Vor, bei und / oder nach der Abtrennung des Fettes ist es vorteilhaft, wenn eine Temperatur unterhalb von 41°C, insbesondere zwischen 37 und 41°C eingestellt wird. Durch die Entfettung entsteht eine Molke.

Die Ausfällung des Kaseins mit dem Labenzym bei Temperaturen findet vorteilhafterweise zwischen 15 und 55°C, insbesondere zwischen 30 und 40°C statt. Wobei es vorteilhaft ist, wenn bei der Ausfällung mit dem Lab-Enzym 10 bis 40 Massen-% der Molke ausgefällt wird und / oder der Proteingehalt auf 4,1 bis 7,1 w/v % eingestellt wird. Durch die Ausfällung des Kaseins entsteht eine sogenannte Sirte.

Es ist vorteilhaft, wenn mindestens eine Grobfiltrationsstufe mit einer mittleren Porengröße zwischen 10 und 100 µm durchgeführt wird. Ferner ist es vorteilhaft, wenn nach der Grobfiltration mindestens eine Vor-Filtrationsstufe mit einem Filter mit einer mittleren Porengröße von 0,2 bis 0,8 µm, insbesondere im Querstromverfahren durchgeführt wird.

Die Filtrationsstufe mit einem Sterilfilter wird vorteilhafterweise mit einer mittleren Porengröße von 0,05 bis 0,3 µm durchgeführt.

Für die Erzielung eines hochqualitativen Produktes ist es vorteilhaft, alle Verarbeitungsschritte ohne Zugabe von Chemikalien und oder Konservierungsstoffen erfolgen.

Bei den Verfahren kann das Rohkolostrum vorteilhafterweise bovinen oder equinen Ursprungs sein oder vom Schaf oder Ziege stammen.

Die Aufgabe wird auch durch eine Vorrichtung zur Durchführung des Verfahrens gelöst. Die Vorrichtung weist Mittel zum Auftauen und mechanischen Zerkleinern gefrorenen Rohkolostrums, ein Mittel zur Abtrennung von Fett, ein Mittel zur Ausfällung von Kasein durch ein Lab-Enzym und mindestens eine Sterilfiltrationsstufe auf. Mit Vorteil weist die Vorrichtung ein Mikrowellenmittel auf, dass beim Auftauen des gefrorenen Rohkolostrums einsetzbar ist.

Im Folgenden werden Aspekte der Erfindung anhand von Ausführungsbeispielen erläutert. Dabei zeigen:
Fig. 1 einen schematischen Verfahrensablauf einer Ausführungsform;
Fig. 2 eine schematische Darstellung einer Querstromfiltration in einer Ausführungsform des Verfahrens;
Fig. 3 eine schematische Darstellung einer Trübungsmessung.

Bei den hier beschriebenen Ausführungsformen wird auf bovines Kolostrum Bezug genommen. Grundsätzlich sind die Ausführungsbeispiele aber auch auf die Herstellung von Kolostrum-Produkten aus Pferde-, Schaf- oder Ziegen-Rohkolostrum anwendbar.

Bei frischem Rohkolostrum handelt es sich um Rohmilch der Kuh direkt nach der Kalbung und wird daher auch "Erstmilch" genannt. Sie besitzt eine hohe Viskosität und eine rötlich gelbe Farbe, hervorgerufen durch rote Blutkörperchen, die im Kolostrum enthalten sind. Die Trockenmasse dieser Milch beträgt ca. 33 Massen-% (im Vergleich: normale Milch ca. 12,5 Massen-%). Der Hauptteil der erhöhten Trockenmasse ist bedingt durch den höheren Eiweißanteil, der 9 bis 22 Massen-% betragen kann.

Die Immunglobuline stellen ca. 60% der Proteine im Kolostrum dar, während in normaler Milch die Kaseine den Hauptanteil der Proteine ausmachen. (siehe z.B. Ternes W.:
Naturwissenschaftliche Grundlagen der Lebensmittelzubereitung, Behr's Verlag, Hamburg, 2. Auflage, 2000)

Dadurch bietet Kolostrum für das neugeborene Kalb eine Schutzfunktion. Für das Kalb ist beispielsweise die Aufnahme von Antikörpern über Kolostrum lebensnotwendig (Terner W., Täufel A., Tunger L., Zobel M.: Lebensmittellexikon, Behr's Verlag, 4. Auflage, 2005). Antikörper sind Immunglobuline, die mit dem Antigen spezifisch reagieren, das ihre Bildung induziert hat.

Die Immunglobuline des Kolostrums schützen vor Infektionen, was besonders wichtig ist, da Kälber nahezu ohne den Schutz von Immunglobulinen geboren werden (siehe Werner A.: Experimentelle Untersuchungen zur Eignung der γ-Glutamyltransferase-Aktivität im Blut von Kälbern zur Überprüfung der Kolostrumversorgung, Inaugural-Dissertation an der Tierärztlichen Hochschule Hannover, 2003).

Immunglobuline kommen als so genannte Abwehrproteine in Blutplasma, Lymphe und vielen Körpersekreten (z.B. Milch aller Wirbeltiere) vor.

In boviner Milch wurden vier Immunglobulinklassen nachgewiesen: IgG, IgA, IgM, IgE. Die Bezeichnung der Immunglobuline wurde entsprechend der von der WHO für humane Immunglobuline eingeführten Nomenklatur für Immunglobuline der Kuh beibehalten.

Alle Immunglobuline weisen ein gemeinsames Bauprinzip auf: Sie bestehen aus mindestens vier Polypeptidketten, von denen jeweils zwei identisch sind. Die als H-Ketten ("heavy", 50000-70000 Da) und L-Ketten ("leichte Ketten", ca. 20000 Da) bezeichneten Strukturen werden kovalent durch Disulfidbrücken verbunden. Die H-Ketten bestehen aus 450-600 Aminosäuren, die L-Ketten aus 211 bis 221- Aminosäuren (siehe Friemel H., Bock J.: Grundlagen der Immunologie, Akademieverlag Berlin, 1986).

Die N-terminalen Hälften beider Ketten enthalten variable Zonen, die für die Antigenbildung verantwortlich sind. Die C-terminalen Bereiche sind hinsichtlich der Aminosäuresequenz sehr konstant und bestimmen die chemischen und physikalischen Eigenschaften dieser Proteinklassen.

Im Kolostrum der Kuh können in den ersten Stunden nach der Geburt des Kalbes Immunglobulinkonzentrationen bis zu 100 g/l erreicht werden. In herkömmlicher Milch liegt der Anteil an Immunglobulinen bei etwa 1 g/l.

Mengenmäßig überwiegen die IgG mit 80-90 Massen-% im Kolostrum. Im ersten Gemelk wurden 2,3 ±1,3 mg/l IgA, 60,5±18,2 mg/l IgG und 10,4±4,1 mg/l IgM gefunden.

Im zweiten Gemelk lagen die Werte bereits deutlich niedriger (IgA 1,2±0,0 mg/ml, IgG 33,3±16,2 mg/l und IgM 5,5±4,0mg/ml (siehe Messerli J.: Messung des klassenspezifischen Antikörper- und Immunglobulingehaltes in Colostrumseren von proteinstimulierten Rindern, Diss. Am Veterinär-Bakteriologischen Inst. Bern 1972).

Wachstumsfaktoren sind Insulin-ähnliche Verbindungen. Man unterscheidet Wachstumsfaktor I (IGF1) und II (IGF2). Beide stimulieren das Zellwachstum. Sie wirken wie endokrine und Hormone über das Blut und lokal als parakrine und autokrine Wachstumsfaktoren. Die Konzentration von IGF1 beträgt 200-2000 µg/l, während in normaler Milch < 10 µg/l.

Rohkolostrum ist hinsichtlich der Hygieneanforderungen vergleichbar mit Vorzugsmilch zu behandeln, die direkt ab Hof abgegeben wird, weder erhitzt noch homogenisiert oder entrahmt ist.

Rohmilch ist jedoch (im Gegensatz zu Kolostrum) ein durch regelmäßiges, vollständiges Ausmelken des Euters sauber gewonnenes Produkt. Dieses Ausmelken des Euters kann bei einer Kuh, die frisch gekalbt hat, für die Gewinnung von Erstmilch nicht erfolgen. Die Versorgung der Bevölkerung mit Rohmilch ist aufgrund der mikrobiellen Belastung (eventuell vorhandenen Krankheitserreger) nicht möglich. Lediglich qualitativ hochwertige Rohmilch von ausgewählten, gesunden Rinderbeständen darf aus diätetischen Gründen für den Frischverzehr im rohen Zustand verwendet werden (siehe Terner W., Täufel A., Tunger L., Zobel M.: Lebensmittellexikon, Behr's Verlag, 4. Auflage, 2005).

Insofern kommt der Qualität von Rohmilch für den menschlichen Verzehr eine große Bedeutung zu, wobei es insbesondere auf Temperatur, Dichte, pH-Wert, Seuchenstatus, Hemmstoffnachweis, SH-Zahl, Sensorik, Zellzahl und Reinheitsgrad ankommt. Kolostrum an sich ist aufgrund seiner mikrobiologischen Beschaffenheit als Rohmilch für den Menschen nicht verzehrbar. Da das Euter der Kuh innerhalb der ersten Stunden gerade mit dem wertvollen Kolostrum gefüllt ist und daher nicht ausgemolken werden kann, ist das Rohkolostrum als infektiös zu bezeichnen. Es ist im flüssigen Zustand frisch von der Kuh nicht lagerfähig.

Die der Sammlung und Lagerung folgenden Aufarbeitungsschritte sind lebensmittelhygienisch zu überwachen. Die zu untersuchenden Parameter orientieren sich an der Schweizer Hygiene-Verordnung: Mesophile Gesamtkeimzahl, Hefen, Schimmelpilze, Staphylococcus aureus, Bacillus cereus, Enterobacteriaceae, sulfitreduzierende Clostridien, Clostridium perfringens, Salmonella ssp., Listeria monocytogenes, Campylobacter jejunii/coli.

Die Reduktion der mikrobiellen Belastung des Kolostrums hat eine große Bedeutung. Eine Milchpasteurisierung, angewendet auf Kolostrum führt jedoch zu einem Verlust von ca. 10 bis 90% Molkenproteine (Jahresbericht der Bundesanstalt für Milchforschung Kiel, Band 35, Heft 2, 1983) oder ca. 58% Immunglobulin (Keech A.M: Peptide Immunotherapy Colostrum a physician's reference guideAKS Publishing, USA 2009), eine UHT-Erhitzung (Ultra high temperature sterilisation) führt zu vollständigem Verlust der IgG in Kolostrum (Keech A.M: Peptide Immunotherapy Colostrum a physician's reference guideAKS Publishing, USA 2009). WO 2009/135306 beschreibt ein Verfahren zur Herstellung einer mit Wachstumsfaktoren angereicherten auf Kolostrum basierenden Fraktion zur Wundheilung und Hautpflege.

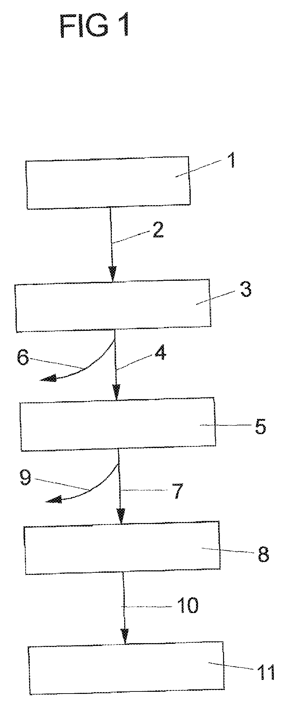

In Fig. 1 wird schematisch eine Ausführungsform des Verfahrens zur Herstellung eines Kolostrum-Produktes dargestellt. Die Einzelheiten der Verfahrensschritte werden dann im Folgenden näher erläutert.

Ausgangsprodukt 1 ist gefrorenes Rohkolostrum, dass in einem Crusher 2 zerkleinert wird. Vorher und / oder parallel dazu erfolgt ein Auftauen.

Das Produkt nach dem Crushen 2 ist flüssiges Rohkolostrum 3. Nach Abtrennung von Fett 4 (z.B. durch Temperierung und Zentrifugation) entsteht eine Molke 5, wobei das Fett 6 abgetrennt wird.

Nach einer Ausfällung von Kasein 7 entsteht eine Sirte 8, wobei Käse 9 als Festprodukt aus dem Verfahren ausgeschleust wird.

Es folgt eine Reihe von Filtrationsschritten 10, wobei mindestens eine Sterilfiltration vorgenommen wird. Das Produkt 11 liegt dann nach der Sterilfiltration vor.

Die Sterilfiltration ist eine Keimabtrennung durch Filtration. Man verwendet bakteriendichte Filtermaterialien mit geringer Porengröße. Diese Art der Entkeimung hat den Vorteil, dass thermolabile Stoff nicht geschädigt werden.

Insofern kommt ein Filtrationsverfahren mit Porengrößen zwischen 0,2 und 14 µm zum Einsatz. Dieses Verfahren wird auch Mikrofiltration genannt. Damit sind Bakterien und größere Viren abtrennbar.

Da Kolostrum aufgrund weiterer Inhaltsstoffe die Porengröße 0,2 µm sofort verstopfen würde, ist ein Filtersystem (Filterkombination) vor der Sterilfiltration vorteilhaft, das gröbere, mittlere und feine Porengrößen aufweist. Ausführungsformen mit vorgeschalteter Grobfiltration und Vorfiltration im Querstrom-Verfahren werden weiter unten angeben.

Als Filtermaterial sind z.B. Glaspulver verschiedener Körnung (z.B. Glassinterfilter), unglasiertes Porzellan (Chamberland-Kerzen), gepresstes Kieselgur (Berkefeld-Filter), Asbest (Seitz-Filter), Membranfilter aus organischen Kolloiden (z.B. Cellulose oder Celluloseester, Kollodium) verwendbar.

Verwendungsbeispiele für diese Filtermaterialen werden weiter unten gegeben. Einige Filtermaterialien sind in Ausführungen mit verschiedener Porengröße erhältlich, mit denen sogar Organismen verschiedener Gestalt und Größe voneinander trennen.

Die einzelne Filtrationsschritte können als statische Filtration (Dead-End-Filtration) oder als dynamische Querstrom-Filtration (auch Cross-Flow-Filtration genannt) durchgeführt werden. Grundsätzlich ist es möglich, dass Filtrationsstufen unterschiedlicher Ausbildung in Kombination miteinander verwendet werden.

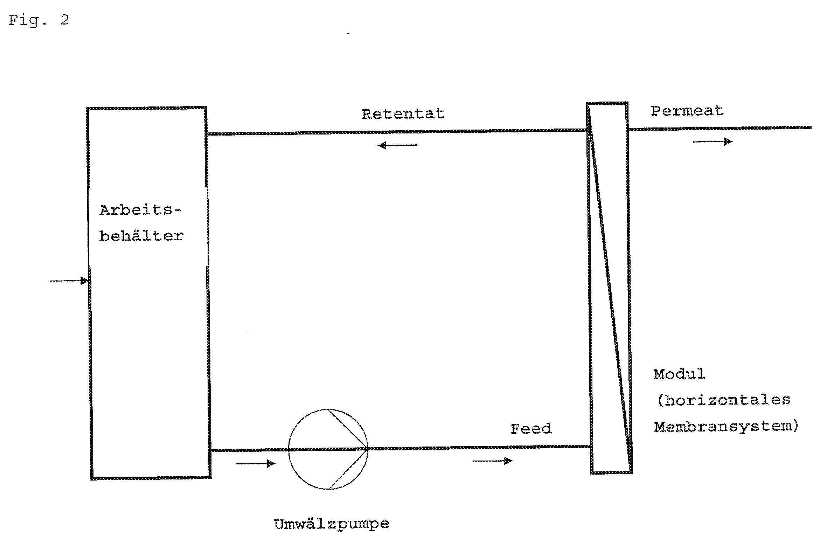

Im Folgenden wird als ein Ausführungsbeispiel eine Vorfiltration mittels Querstrom-Filtration beschrieben (siehe auch Fig. 2). Dabei wird das zu filtrierende Gut (d.h. das Rohkolostrum), das einem Arbeitsbehälter zugeführt wird, horizontal mittels einer Umwälzpumpe durch einen Konzentratkanal eines Membranmoduls zirkuliert. Das Permeat (bei der Dead-End-Filtration Filtrat genannt) tritt über porösen Membranen aus dem Membranmodul aus, während das Retentat permanent zirkuliert wird, bis die Trübstoffe im Retentat so hoch konzentriert sind, dass eine Spülung bzw. Reinigung der Anlage erforderlich ist.

Aufgrund des angelegten Druckes und damit der laminaren Strömung über das Strömungsprofil werden durch das Überströmen des Retentates auf den Membranen Scherkräfte erzeugt. Diese Scherkräfte verhindern die Ausbildung von Rückständen oder Filmen (Deckschicht oder Fouling) auf der Innenseite der Membranen.

Die Auswahl der Membranmaterialien ergibt sich aus der Filtrationsaufgabe. Anorganische Filtrationsmaterialien kommen zum Einsatz, wo höhere thermische und/oder chemische Beständigkeit erforderlich ist. Anorganische Materialien sind z.B. keramische von metallischen und kristallinen Membranen. Überall dort, wo die Lebensdauer von Polymermembranen ausreichend ist kommen organische Materialien zum Einsatz. Es kann sich hierbei um Einzelpolymere handeln, um Kombinationen verschiedener Polymere oder um funktionale Polymere, d.h. oberflächenmodifizierte Membranen für bestimmte adsorptive Prozesse oder katalytische Wirkungen. Zu den Einzelpolymeren zählen z.B. Polypropylen, Polysulfon, Polyethersulfon, Teflon, etc. Mischpolymere sind z.B. Polyester/Polyamid, Polyester/PSU.

Die Anforderungen an die Aufarbeitung von Rohkolostrum können wie folgt zusammengefasst werden:
1. Herstellung eines bei Raumtemperatur (ca. 20 °C) über ca. 12 Monate lagerfähigen flüssigen Kolostrums.
2. Erhalt der wertbestimmenden Kolostrum-Inhaltsstoffe unter schonender thermischer Behandlung.
3. Herstellung eines Kolostrums mit möglichst geringer Keimzahl bei gleichzeitiger Abwesenheit von pathogenen Keimen.
4. Herstellung eines Getränkes mit möglichst hoher Transparenz bzw. geringer Trübung.

Im Folgenden werden relevante Schritte einer Ausführungsform des Verfahrens zur der Herstellung eines Kolostrum-Produktes beschrieben. Für den Fachmann ist klar, dass nicht alle diese Schritte zwingend zusammen ausgeführt werden müssen. Es ist auch möglich, einzelne Schritte wegzulassen oder andere Kombinationen von Verfahrensschritten zu verwenden.

Die Sammlung von Rohkolostrum erfolgt im Kuhstall. Die Sammlung umfasst z.B. die Erstmilch von laktierenden Kühen. Gesammelt wird die Milch der ersten 0 bis 12 Stunden nach der Kalbung (1. Melkung) und aus dem Zeitraum 12 bis 24 Stunden (2. Melkung). In diesen Zeiträumen ist die Sammlung von Rohkolostrum besonders vorteilhaft, da der Anteil der Immunglobuline im Laufe der Zeit abfällt.

Blutiges Kolostrum wird verworfen. Das Kolostrum wird chargenrein (d.h. keine Mischung von Milch unterschiedlicher Kühe) in wieder verwendbaren Sammelgefäßen aus Kunststoff eingefroren. Vom Stall wird das gefrorene Kolostrum unter Einhaltung der Kühlkette transportiert und im Kolostrum-Verarbeitungsbetrieb einer Wareneingangskontrolle unterzogen. Aus jedem Sammelbehälter werden Proben gezogen. Es erfolgt eine visuelle Prüfung (z.B. wird blutiges Kolostrum verwerfen, Prüfung auf mikrobiologische Parameter, Hemmstoffe (Antibiotika), Immunglobuline (IgG). Die Lagerung erfolgt bei -18 °C im Kühllager.

Im Weiteren wird das Rohkolostrum einer Reihe von Verarbeitungsschritten unterzogen, von denen einige im Folgenden beschrieben werden.

Rohkolostrum ist von seiner mikrobiologischen Beschaffenheit ein äußerst sensibler Rohstoff. Das tiefgefrorene Rohkolostrum muss aufgetaut werden und sollte bezüglich der Temperaturbelastung eine geringstmögliche Belastung erfahren.

Bei bekannten Verfahren wird die tiefgefroren Kolostrumware über Nacht bzw. bis zu 2 Tagen (ca. 12 bis 48 Stunden aufgetaut) nimmt Raumtemperatur an und wird dann zur Entfettung aufgeheizt. Die im Rohkolostrum enthaltenen Keime unterliegen über diesen Zeitraum einer erheblichen Vermehrungsrate zu Lasten des Herstellprozesses und der Qualität des Endproduktes.

Im hier beschriebenen Verfahren wird das gefrorene Rohkolostrum in einen Eisbrecher (Icecrusher) verbracht.

Die kontinuierliche Vermahlung des gefrorenen Kolostrum-Eises zu Eispartikeln mit einem Durchmesser von ca. 1 cm dauert ca. 5 bis 10 Sekunden pro Block von 3 kg, wobei ca. 6 bis 7 Blöcke in ca. 15 bis 18 Sekunden gleichzeitig bearbeitet werden. Die durchschnittliche Geschwindigkeit beträgt ca. 2,5 s/kg.

Gleichzeitig oder danach kann das granulierte Kolostrum-Eis direkt kontinuierlich in einen Mischer verbracht werden, der über eine Thermostatisierung und gleichzeitiges Mischen die Temperatur des Rohkolostrums auf weniger als 10 °C, insbesondere zwischen 4 bis 10 °C für die Weiterverarbeitung einstellt.

Beim Auftauen findet eine Phasentrennung zwischen dem festen (noch gefrorenen) und dem bereits geschmolzenen Kolostrum bei einer minimalen thermischen Belastung statt. Das geschmolzene Kolostrum wird kontinuierlich abgezogen und kann im nächsten Verfahrensschritt einer Entfettung unterzogen werden. Der Vorteil dieses Verfahrens ist, dass das Rohkolostrum deutlich geringere Keimzahlen in den Verarbeitungsprozess mitbringt als mit dem herkömmlichen Verfahren und die wertbestimmenden Inhaltsstoffe (IgG und IgF1) weitestgehend erhalten bleiben. Zum Nachweis der Keimzahlminimierung durch das Crushen werden im Vergleich zwei Ansätze
**A:** Crushen bei -18°C/
**B:** 24h bei 20°C Auftauen, anschließend Crushen)
parallel aus gleichen Lieferquellen parallel behandelt. Die Grundbelastung des unaufgetauten Rohkolostrums lag zwischen 14 bis 62 Mill. KBE/g (Probennahme an der Oberfläche).

Ansatz A: Wurde unmittelbar nach Entnahme aus der Tiefkühlkammer gecrusht und in einem Mischer und Rührer der Fa. Hebold auf +6°C gebracht. Unmittelbar nach crushen und auftauen lag die Gesamtkeimzahl bei 9,5 x 10⁷ KBE/g. Bei Lagerung dieses Rohkolostrums über 48 und 96 Stunden steigt die Gesamtkeimzahl über 18,4x10⁷ auf 37 x10⁷ KBE/g.

Ansatz B war nach 24 h bei 20°C Raumtemperatur nur teilweise in den Behältern aufgetaut, die aus dem Überstand entnommene Probe lag bei 4,5 Mill. KBE/g Vollständig aufgetaut waren die Behälter nach 48 h (zweiter Tag bei 5°C oder 20°C). Proben aus dem komplett aufgetauten Kolostrum dieser Ansätze lagen nach 24 h bei 4,5 x 10⁷ KBE/g und steigerte sich auf 1,25 x 10⁹KBE/g nach 48 h und 1,60 x 10⁹ KBE/g nach 96 h (siehe Tabelle 1).

Langsames Vorgehen bis zum vollständigen Durchtauen des Rohkolostrums führte zu deutlich erhöhten Keimzahlen, und zwar unabhängig davon ob die Behälter bei 20°C oder bei 5°C gelagert wurden, während gecrushtes Rohkolostrum während des selben Lagerzeitraumes erheblich geringer belastet bleibt.

Daraus ist zu schließen, dass die Kombination von Eiscrushen mit dem Auftauprozess im Gegensatz zu langsamen Auftauen zu einem deutlich geringeren Risiko bzgl. Keimbelastung führt.

Als Vorbereitung für die Entfettung wird das aufgeschmolzene Kolostrum vorteilhafterweise zur Aufrahmung in einem temperierbaren Behälter aufgewärmt. Die Temperatur liegt zwischen 37 und 41°C.

Danach erfolgt die Entfettung durch eine Zentrifugation (z.B. in einer Milchzentrifuge) statt. Dadurch wird der Fettgehalt von ca. 10 Massen-% auf weniger als 0,5 Massen-% eingestellt.

Zusätzlich oder alternativ können auch andere Trennschritte zur Entfettung, wie z.B. eine Sedimentation eingesetzt werden. In jedem Fall entsteht eine nicht-sterile Molke.

Anschließend erfolgt vorteilhafterweise eine erste Filtration zur Entlastung der folgenden Sterilfiltration, insbesondere einer Steril-Querstrom-Filtration mit einer Porengröße von ca. 40 µm.

Die Entkaseinierung von Kolostrum nach der Entfettung entspricht einer Fraktionierung von Milch. Hierzu wird die entfettete Kolostrum-Molke einem Fällungsverfahren unterzogen. Dieser Fällvorgang entspricht weitestgehend der Kaseinherstellung bei Schnittkäse.

Der Unterschied beruht darauf, dass bei der Schnittkäseherstellung das ausgefällte Labkasein zum Käse verarbeitet wird und die verbleibende Molke ein "Abfallprodukt" darstellt.

Im vorliegenden Verfahren zur Herstellung eines Kolostrum-Produktes entsteht eine hochwertige Molke mit einem hohen Anteil an Immunglobulinen. Der durch Lab das ausgefällte Käse ist ein Abfallprodukt, das aufgrund der mikrobiologischen Beschaffenheit ohne weite Be- oder Verarbeitung für den menschlichen Verzehr ungeeignet ist.

Die Labfermentation wird initiiert durch Zugabe des Enzyms Chymosin. Bei Chymosin (auch EC 3.4.23.4 oder "Rennin" oder "Labferment" genannt) handelt es sich um eine Protease (proteolytisches Enzym), das spezifisch eine Methionyl-Phenylalanylbindung im Kasein spaltet unter Bildung von Para-k-Kasein und Caseinmakropeptid (CMP).

Beim Dicklegen der Milch spaltet das Labenzym selektiv die Peptidbindung zwischen den Aminosäuren Phenylalanin und Methionin im k-Kasein-Molekül. Dabei entstehen para-k-Casein und ein Glycopeptid, einem Peptid mit einem Kohlenhydratanteil. Während das Glycopeptid löslich ist, wird para-k-Casein durch Ca2+-Ionen der Milch fällbar. Die ursprüngliche Schutzwirkung des k-Kaseins geht also durch die Peptidspaltung verloren und es kommt zur Ausfällung der Kaseinkomplexe und Kaseinmicellen, die Milch wird "dick". Da keine Säuerung erfolgt, bleibt die Milch süß.

Durch die Labspaltung wird die wasserlösliche zum Teil kohlenhydrathaltige Kasein-Teilstruktur CMP freigesetzt. Dieser Vorgang führt in der Milch zur Ausfällung des restlichen Kaseins (Terner W., Täufel A., Tunger L., Zobel M.: Lebensmittellexikon, Behr's Verlag, 4. Auflage, 2005). Chymosin wird aus dem vierten Magen von Kälbern durch saure Extraktion und anschließende Aufreinigung gewonnen. Es spaltet das Kaseineiweiß der Milch an einer bestimmten Stelle und bewirkt dadurch deren Gerinnung. "Dicklegung". Das Kasein verklumpt und trennt sich von der wässrigen Molke.

Die Dicklegung mit Lab erfolgt annähernd im neutralen Bereich. Das Temperaturoptimum der Enzymbehandlung liegt sich zwischen 15 und 55 °C idealerweise bei 41°C.

Bei der Ausfällung mit dem Lab-Enzym wird 10 bis 40 Massen-% des Kaseins ausgefällt und / oder der Proteingehalt auf 4,1 bis 7,1 w/v % (Gewicht/Volumen-%: Der Anteil des ausgefällten Kaseins wird ausgewogen und auf das Volumen des Kolostrums bezogen.) eingestellt.

Nach der Ausfällung des Kaseins erfolgt mindestens ein Sterilfiltrationsschritt.

Dies wird in der Regel aber nicht der einzige Filtrationsschritt sein; typischerweise sind der Sterilfiltration einer oder mehrere Filtrationen vorgeschaltet.

In einer vorteilhaften Ausführungsform erfolgt nach der Ausfällung des Kaseins zuerst eine Grobfiltration mittels eines Schneckenseparators. Typischerweise liegt dabei eine mittlere Porengröße zwischen 10 und 100 µm vor.

Anschließend ist es vorteilhaft, wenn eine Vorfiltration, z.B. mittels einer Querstrom-Filtration (cross-flow filtration) erfolgt. Bei einer Querstrom-Filtration existiert eine Flüssigkeitsströmung parallel zur Filterfläche. In der Regel erfolgt eine solche Querstrom-Filtration in Membranenmodulen.

Bei dieser Vorfiltration kommen Filter mit mittleren Porengrößen zwischen 0,2 und 0,8 µm zum Einsatz. Es wird mindestens eine Vor-Filtrationsstufe mit einem Tiefenfilter der mittleren Porengröße 0,65 µm durchgeführt. Dieses Vorfilterelement besteht z.B. aus PES mit einer Nylon-Filtermembran und Polypropylen-Stützfliese. Der Vorfilter kann alternativ auch als Glasfilter (mittlere Porengröße 0,65 µm), Polypropylen (mittlere Porengröße 0,65 µm oder 0,5 µm), Polyethersulfon oder PES (mittlere Porengröße 0,65 oder 0,8 µm) ausgebildet sein.

Schließlich erfolgt die eigentliche Sterilfiltration, bei der die mittlere Porengröße ca. zwischen 0,05 und 0,3 µm beträgt. Diese Sterilfiltration kann in Form eines Absolutkerzenfilters, insbesondere mit einem mittleren Porendurchmesser von 0,2 µm durchgeführt werden. Das Material des Sterilfilters ist PES, Die Filtermembran besteht aus Nylon und die Vliese und Stützkörper aus PP. Alternativ kann verwendet werden ein Endfilter PES als Doppelfilter der Porengrößen 0,35/0,2 µm.

Inhaltsstoffe wie Wachstumsfaktoren (IgF-1, EgF, TgF, etc.), Immunglobuline (IgG, IgA, IgM, etc.), Aminosäuren, organische Moleküle (Kreatinin, Lactoferrin, etc.) und Vitamine bleiben erhalten, da sie die selektive Membran der Sterilfiltration passieren.

Das Produkt ist während des Prozesses nicht der Umgebungsluft ausgesetzt zur Vermeidung einer Rekontamination.

Die einzelnen Prozessschritte sind manuell und / oder automatisiert reinigungs- und sterilisierbar.

Die Abfüllung des flüssigen Kolostrums-Produktes erfolgt z.B. in sterilisierten Glasflaschen oder in sterilen Kunststoffflaschen.

Damit liegt ein flüssiges Kolostrum-Produkt mit neuen Eigenschaften vor, das bei Raumtemperatur haltbar und nicht pasteurisiert ist.

Ausgehend von einem hochbelasteten Rohkolostrummaterial (entfettet und entkaseiniert) mit einer Gesamtkeimzahl von mehr als 3 x 10⁶ cfu/g (cfu/g wird auch als KBE/g angegeben) wird durch das beschriebene Verfahren eine Gesamtkeimzahl von weniger 10 cfu/g im Permeat erzielt, während im Retentat die Keimbelastung erwartungsgemäß bei mehr 3x10⁵ cfu/g blieb.

Ebenso der Gehalt an Hefen, Schimmelpilzen, Staphylococcus aureus, Bacillus cereus, Enterobacterieceen, sulfitreduzierende Clostridien, Salmonellen und Listerien sowie Campylobacter im Permeat konnten durch das beschriebene Verfahren auf weniger als 10 cfu/g reduziert werden.

Das beschriebene Verfahren hat keine Auswirkungen auf den Gehalt an IgG, IgF1 und Lactose im Kolostrum-Produkt. Der Lactosegehalt im Rohkolostrum (entfettet und entkaseiniert) beträgt ca. 2,8% vor der Sterilfiltration und im Permeat.

Ausgehend von einem IgG-Gehalt von 34 g/l im Rohkolostrum (entfettet und entkaseiniert) enthält das gewonnene, sterilfiltrierte Kolostrum-Produkt unverändert 34 g/l IgG.

Auch der IgF1-Gehalt im Rohkolostrum (entfettet und entkaseiniert) von ursprünglich 123 ng/ml wird durch die erfolgte Sterilfiltration nicht beeinträchtigt und blieb trotz Sterilfiltration konstant im Permeat.

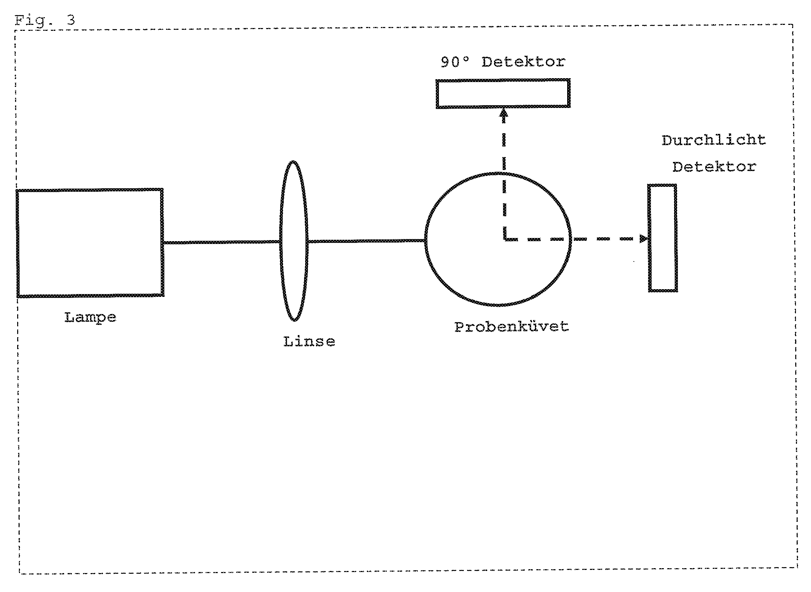

Das beschriebene Verfahren erzielt ein Produkt mit äußerst geringer Trübung. Bei herkömmlich hergestellten kolostralen Getränken sind Trübungen im Bereich von 180 FNU oder mehr messbar. Bei dem vorliegenden Verfahren sind Trübungen von 50 bis 100 FNU erzielbar. Die Messung der Trübung erfolgt mit einem Trübungsmessgerät nach dem Prinzip der nephelometrischen Trübungsmessung. Das Lichtwegdiagramm (siehe Fig. 3) dieses Gerätes umfasst eine LED-Lampe, einen 90° Detektor zur Streulichtüberwachung sowie einen Durchlicht-Detektor. Der Mikroprozessor des Gerätes berechnet das Verhältnis der der Signale der 90°- und der Durchlicht-Fotozellen. Diese Verhältnistechnik gleicht Interferenzen durch Farbe und/oder Licht absorbierende Materialien (wie Aktivkohle) aus und kompensiert Schwankungen in der Lampenintensität, woraus langfristige Kalibrierstabilität resultiert. Durch diese optische Konstruktion wird außerdem Streulicht minimiert, was wiederum die Messgenauigkeit erhöht.

Das beschriebene Verfahren erzielt ein Produkt mit vollständig erhaltenem Gehalt an Spurenelementen Mangan, Nickel und Silizium sowie Fructose, Saccharose und Galactose.
Es konnte ein Mangangehalt von 8 mg/l Kolostrum festgestellt werden. Der Körperbestand des erwachsenen Menschen beträgt ca. 12 bis 20 mg. Mangan befindet sich überwiegend im Knochenmark, in Leber, Nieren und in der Bauchspeicheldrüse. Die Schätzwerte der Deutschen Gesellschaft für Ernährung für Jugendliche und Erwachsene betragen 2 bis 5 mg/Tag. Mangan benötigt der Mensch, da es integraler Bestandteil verschiedener Metalloenzyme ist, die bei der Glucogeonese, im Glutaminstoffwechsel und Harnstoffzyklus von Bedeutung sind. Auch das Enzym Superoxiddismutase ist manganabhängig. Mangan wird bei einer Reihe von enzymatischen Reaktionen als Cofaktor verwendet.

Im Folgenden wird eine weitere Ausführungsform beschrieben, deren einzelne Verfahrenschritte sich auch in den vorherigen Ausführungsformen einsetzen lassen.
1. Das Rohkolostrum wird in den 3 Liter-Behältern angetaut und in einen Crusher gegeben. Dieser Verfahrensschritt kann pro Kilogramm z.B. 1 bis 5 Sekunden dauern. Wenn andere Behältergrößen verwendet werden, kann die Verfahrensdauer dieses Schrittes u.U. angepasst werden.
2. Anschließend wird die zerkleinerte (gecrushte) Eismasse zu einem doppelwandigen Behälter transportiert und unter konstantem Rühren auf ca. 4 bis 5 °C erwärmt. Dieser Verfahrensschritt kann z.B. 10 bis 12 Minuten dauern.
3. Anschließend erfolgt die Abfüllung in einen gekühlten Tank, in dem der Inhalt bei maximal 6°C gelagert wird.
4. Abfüllung einer Charge (z.B. 2 x 150 Liter oder 2 x 500 Liter) in einen temperierbaren (z.B. doppelwandigen) Behälter zur Erhitzung auf ca. 38°C. Im Folgenden wird beispielhaft von je zwei 150 Liter Behältern ausgegangen,
5. Anschließend erfolgt eine Entfettung z.B. mit einer Milchzentrifuge.
6. Dann wird das Lab-Enzym Chymosin zwei 150 Liter Behälter (temperierbar) zum entfetteten Kolostrum gegeben. Die Temperatur beträgt z.B. 33 bis 38°C.
7. Nach ca. 1 Stunde kann das ausgefällte Kasein zerschnitten werden. Dies erhöht die Oberfläche und vervollständigt die Entkaseinierung.
8. Entnahme des Kaseins und / oder Abschöpfen aus der Molke nach frühestens ca. 2 Stunden. Vorteilhafterweise nach 12 bis 18 Stunden.
9. Grobreinigung der Molke durch Kaskadenfilter (Schneckenseparator) über Schwerkraft. Die Kaskadenfilter haben z.B. Netzgrößen 100 µm, 50 µm und 10 µm.
10. Es ist vorteilhaft, wenn das Produkt unmittelbar der Vorfiltration mittels einer Querstromfiltration unterzogen wird.
11. Anschließend erfolgt dann die Sterilfiltration.

Insbesondere mit den hier beschriebenen Ausführungsformen des Herstellungsverfahrens ist ein Kolostrum-Produkt herstellbar, das eine Gesamtkeimzahl von weniger als 100 cfu/g, insbesondere weniger als 10 cfu/g aufweist. Außerdem weist das Kolostrum-Produkt eine geringe Trübung mit Werten von 50 bis 100 FNU auf. Auch ist es möglich, dass das Kolostrum-Produkt einen Mangangehalt von 5 bis 15 mg/l aufweist. Der Nickelgehalt des erhaltenen entfetteten und entkaseinierten, sterilfiltriertem Kolostrums enthält 5 bis 10 mg/l Nickel und 50 bis 100 mg/l Silizium. Der Fructosegehalt beträgt im Fertigprodukt 5 bis 50 mg/l, Saccharosegehalt 0 bis 15 mg/l und Galactosegehalt 1 bis 2 g/l. Eine besonders vorteilhafte Ausführungsform des Verfahrens weist die folgenden Schritte auf:
a) Gefrorenes Rohkolostrum auf eine Temperatur unterhalb von 10 °C, vorzugsweise 4 bis 10°C aufgetaut, wobei anschließend
b) durch eine Abtrennung ein Fettgehalt von weniger als 0,5% Massen-%, insbesondere von weniger als 0,2 Massen-% eingestellt wird, und dann
c) eine Ausfällung von Kasein durch eine Zugabe einer Protease in Form von des Lab-Enzyms Chymosin erfolgt und
d) die erhaltene Flüssigkeit mindestens einer Sterilfiltrationsstufe unterzogen wird.

Dieses Kolostrum-Produkt kann als Getränk, Lebensmittel, insbesondere auch diätetischem Lebensmittel, klinischer Nahrung, Sondennahrung, Babynahrung, Nahrungsergänzungsmittel oder Arzneimittel verwendet werden.

Das Kolostrum-Produkt kann auch als Zutat zur Herstellung von Lebensmitteln, insbesondere auch diätetischem Lebensmittel, klinischer Nahrung, Sondennahrung, Babynahrung, Nahrungsergänzungsmitteln oder Arzneimitteln verwendet werden.

**Tabelle 1**

| **Homogenat** | **Zeit** | **Nach Antauen 24h bei 20°C** | **Nach Antauen 48h bei 5°C** | **Nach Antauen 96h bei 5°C** |
|---|---|---|---|---|
| **Ansatz A** | Nach Crushen auftauen | | | |
| **Gesamtkeimzahl (KBE/g)** | | 9.500.000 | 18.400.000 | 37.000.000 |
| **Ansatz B** | langsames Auftauen | | | |
| **Gesamtkeimzahl (KBE/g)** | | 4.500.000 | 125.000.000 | 160.000.000 |

## Patentansprüche

1. Verfahren zur Herstellung eines Kolostrum-Produktes, wobei
a) gefrorenes Rohkolostrum aufgetaut und auf eine Temperatur unterhalb von 10 °C, vorzugsweise 4 bis 10°C aufgetaut wird, wobei das gefrorene Rohkolostrum vor und / oder während des Auftauens mechanisch in kürzester Zeit zerkleinert wird, und anschließend
b) durch eine Abtrennung ein Fettgehalt von weniger als 0,5 Massen-%, insbesondere von weniger als 0,2 Massen-% eingestellt wird, und dann
c) eine Ausfällung von Kasein durch eine Zugabe einer Protease in Form von des Lab-Enzyms Chymosin erfolgt und
d) die erhaltene Flüssigkeit mindestens einer Sterilfiltrationsstufe unterzogen wird.

2. Verfahren nach Anspruch 1, **dadurch gekennzeichnet, dass** das Auftauen des gefrorenen Rohkolostrums innerhalb von 1 bis 5 Sekunden pro kg erfolgt.

3. Verfahren nach mindestens Anspruch 1 oder 2, **dadurch gekennzeichnet, dass** vor und / oder während des Auftauens eine kontinuierliche Vermahlung des gefrorenen Rohkolostrum-Eises zu Eispartikeln mit einem mittleren Durchmesser von ca. 1 cm erfolgt.

4. Verfahren nach mindestens einem der vorhergehenden Ansprüche, **dadurch gekennzeichnet, dass** mechanische Zerkleinern zwischen 1 bis 5 Sekunden pro kg dauert, ggf. kontinuierlich stattfindet, wobei das aufgetaute Rohkolostrum kontinuierlich abgezogen wird.

5. Verfahren nach mindestens einem der vorhergehenden Ansprüche, **dadurch gekennzeichnet, dass** das gefrorene Rohkolostrum zumindest teilweise während der mechanischen Zerkleinerung aufgetaut wird.

6. Verfahren nach mindestens einem der vorhergehenden Ansprüche, **dadurch gekennzeichnet, dass** vor, bei und / oder nach der Abtrennung des Fettes eine Temperatur unterhalb von 41°C, insbesondere zwischen 37 und 41°C eingestellt wird.

7. Verfahren nach mindestens einem der vorhergehenden Ansprüche, **dadurch gekennzeichnet, dass** die Einstellung des Fettgehaltes durch Zentrifugieren, insbesondere eine Milchzentrifuge, eine Schneckenzentrifuge und / oder Sedimentieren erreicht wird.

8. Verfahren nach mindestens einem der vorhergehenden Ansprüche, **dadurch gekennzeichnet, dass** die Ausfällung des Kaseins mit dem Labenzym bei Temperaturen zwischen 15 und 55°C, insbesondere zwischen 30 und 40 stattfindet.

9. Verfahren nach mindestens einem der vorhergehenden Ansprüche, **dadurch gekennzeichnet, dass** bei der Ausfällung mit dem Lab-Enzym 10 bis 40 Massen-% des Molke ausgefällt wird und / oder der Proteingehalt auf 4,1 bis 7,1 w/v % eingestellt wird.

10. Verfahren nach mindestens einem der vorhergehenden Ansprüche, **dadurch gekennzeichnet, dass** mindestens eine Grobfiltrationsstufe mit einer mittleren Porengröße zwischen 10 und 100 *µ*m durchgeführt wird.

11. Verfahren nach mindestens einem der vorhergehenden Ansprüche, **dadurch gekennzeichnet, dass** mindestens eine Vor-Filtrationsstufe mit einem Filter mit einer mittleren Porengröße von 0,2 bis 0,8 *µ*m, insbesondere im Querstromverfahren durchgeführt wird.

12. Verfahren nach mindestens einem der vorhergehenden Ansprüche, **dadurch gekennzeichnet, dass** mindestens eine Filtrationsstufe mit einem Sterilfilter, mit einer mittleren Porengröße im Bereich von 0,05 bis 0,3 *µ*m durchgeführt wird.

13. Verfahren nach mindestens einem der vorhergehenden Ansprüche, **dadurch gekennzeichnet, dass** alle Verarbeitungsschritte ohne Zugabe von Chemikalien und oder Konservierungsstoffen erfolgen.

14. Verfahren nach einem der vorhergehenden Ansprüche, **dadurch gekennzeichnet,** das Rohkolostrum bovinen oder equinen Ursprungs ist oder vom Schaf oder Ziege stammt.

15. Vorrichtung zur Durchführung mindestens eines Verfahrens gemäß der Ansprüche 1 bis 15, **gekennzeichnet durch** Mittel zum Auftauen und mechanischen Zerkleinern gefrorenen Rohkolostrums, einem Mittel zur Abtrennung von Fett, einem Mittel zur Ausfällung von Kasein **durch** ein Lab-Enzym und mindestens eine Sterilfiltrationsstufe, wobei insbesondere ein Mikrowellenmittel beim Auftauen des gefrorenen Rohkolostrums einsetzbar ist.

## Claims

1. Method for manufacturing a colostrum product, wherein
a) frozen raw colostrum is thawed and thawed to a temperature below 10°C, preferably 4 to 10°C, wherein the frozen raw colostrum before and/or during the thawing is mechanically comminuted in a very short time, and then
b) by a separation, a fat content of less than 0.5 mass%, in particular less than 0.2 mass%, is established, and then
c) a precipitation of casein proceeds by an addition of a protease in the form of the rennet enzyme chymosin and
d) the liquid obtained is subjected to at least one sterile filtration stage.

2. Method according to Claim 1, **characterized in that** the frozen raw colostrum is thawed within 1 to 5 seconds per kg.

3. Method according to at least Claim 1 or 2, **characterized in that** before and/or during the thawing, the frozen raw colostrum ice is continuously milled to form ice particles having a median diameter of approximately 1 cm.

4. Method according to at least one of the preceding claims, **characterized in that** mechanical comminution lasts between 1 to 5 seconds per kg, or takes place continuously, wherein the thawed raw colostrum is taken off continuously.

5. Method according to at least one of the preceding claims, **characterized in that** the frozen raw colostrum is thawed at least in part during the mechanical comminution.

6. Method according to at least one of the preceding claims, **characterized in that** before, during and/or after the fat is separated off, a temperature below 41°C, in particular between 37 and 41°C, is established.

7. Method according to at least one of the preceding claims, **characterized in that** the fat content is established by centrifugation, in particular a milk centrifuge, a screw centrifuge and/or sedimentation.

8. Method according to at least one of the preceding claims, **characterized in that** the casein is precipitated by the rennet enzyme at temperatures between 15 and 55°C, in particular between 30 and 40.

9. Method according to at least one of the preceding claims, **characterized in that** during the precipitation by the rennet enzyme, 10 to 40 mass% of the whey is precipitated out and/or the protein content is established at 4.1 to 7.1 w/v%.

10. Method according to at least one of the preceding claims, **characterized in that** at least one coarse filtration stage having a median pore size between 10 and 100 µm is carried out.

11. Method according to at least one of the preceding claims, **characterized in that** at least one pre-filtration stage is carried out with a filter having a median pore size from 0.2 to 0.8 µm, in particular in the cross-flow method.

12. Method according to at least one of the preceding claims, **characterized in that** at least one filtration stage is carried out using a sterile filter having a median pore size in the range from 0.05 to 0.3 µm.

13. Method according to at least one of the preceding claims, **characterized in that** all processing steps proceed without addition of chemicals and/or preservatives.

14. Method according to any one of the preceding claims, **characterized in that** the raw colostrum is of bovine or equine origin, or originates from sheep or goats.

15. Device for carrying out at least one method according to Claims 1 to 15, **characterized by** means for thawing and mechanical comminution of frozen raw colostrum, a means for separating off fat, a means for precipitating casein by a rennet enzyme and at least one sterile filtration stage, wherein in particular a microwave means is usable in the thawing of the frozen raw colostrum.

## Revendications

1. Procédé de fabrication d'un produit de colostrum, dans lequel
a) on décongèle du colostrum brut congelé, et on le décongèle à une température inférieure à 10°C, de préférence de 4 à 10°C, le colostrum brut congelé étant, avant et/ou pendant la décongélation, soumis à un broyage mécanique en un temps extrêmement court, puis
b) sous l'effet d'une séparation, on ajuste la teneur en matière grasse à une valeur inférieure à 0,5 % en masse, en particulier inférieure à 0,2 % en masse, puis
c) on procède à une précipitation de caséine par addition d'une protéase sous forme de l'enzyme de présure chymosine, et
d) on soumet le liquide obtenu au moins à une étape de filtration dans des conditions stériles.

2. Procédé selon la revendication 1, **caractérisé en ce que** la décongélation du colostrum brut congelé a lieu en un laps de temps de 1 à 5 secondes par kg.

3. Procédé selon au moins la revendication 1 ou 2, **caractérisé en ce qu'**on procède, avant et/ou pendant la décongélation, à un broyage continu de la glace de colostrum brut congelé, pour obtenir des particules de glace ayant un diamètre moyen d'environ 1 cm.

4. Procédé selon au moins l'une des revendications précédentes, **caractérisé en ce que** le broyage mécanique dure de 1 à 5 secondes par kg, éventuellement a lieu en continu, le colostrum brut décongelé étant prélevé en continu.

5. Procédé selon au moins l'une des revendications précédentes, **caractérisé en ce que** le colostrum brut congelé est au moins partiellement décongelé pendant le broyage mécanique.

6. Procédé selon au moins l'une des revendications précédentes, **caractérisé en ce que**, avant, pendant et/ou après la séparation de la matière grasse, on ajuste la température à une valeur inférieure à 41°C, en particulier de 37 à 41°C.

7. Procédé selon au moins l'une des revendications précédentes, **caractérisé en ce que** l'ajustement de la teneur en matière grasse est réalisé par centrifugation, en particulier par une centrifugeuse à lait, une centrifugeuse à vis sans fin et/ou une sédimentation.

8. Procédé selon au moins l'une des revendications précédentes, **caractérisé en ce que** la précipitation de la caséine a lieu avec l'enzyme de présure à des températures comprises entre 15 et 55°C, en particulier entre 30 et 40.

9. Procédé selon au moins l'une des revendications précédentes, **caractérisé en ce que**, lors de la précipitation avec l'enzyme de présure, on provoque la précipitation de 10 à 40 % en masse du sérum, et/ou on ajuste la teneur en protéines à une valeur de 4,1 à 7,1 p/v %.

10. Procédé selon au moins l'une des revendications précédentes, **caractérisé en ce qu'**on met en oeuvre une étape de filtration grossière, avec une grosseur moyenne de pore comprise entre 10 et 100 µm.

11. Procédé selon au moins l'une des revendications précédentes, **caractérisé en ce qu'**on met en oeuvre au moins une étape de préfiltration avec un filtre ayant une grosseur moyenne de pore de 0,2 à 0,8 µm, en particulier par le procédé à courant transversal.

12. Procédé selon au moins l'une des revendications précédentes, **caractérisé en ce qu'**on met en oeuvre au moins une étape de filtration avec un filtre stérile, ayant une grosseur moyenne de pore comprise dans la plage de 0,05 à 0,3 µm.

13. Procédé selon au moins l'une des revendications précédentes, **caractérisé en ce que** toutes les étapes du traitement sont réalisées sans addition de produits chimiques et/ou de conservateurs.

14. Procédé selon l'une des revendications précédentes, **caractérisé en ce que** le colostrum brut est d'origine bovine ou équine, et provient d'une brebis ou d'une chèvre.

15. Dispositif pour la mise en oeuvre d'au moins un procédé selon les revendications 1 à 15, **caractérisé par** des moyens pour la décongélation et le broyage mécanique du colostrum brut congelé, un moyen pour séparer la matière grasse, un moyen pour provoquer la précipitation de la caséine par un enzyme de présure, et au moins une étape de filtration dans des conditions stériles, au moins un moyen de micro-onde étant utilisable lors de la décongélation du colostrum brut congelé.
